# EUROPEAN PATENT APPLICATION

(11) **EP 4 834 322 A1**
(43) Date of publication of application: **04.11.2026**
(21) Application number: 24913649.0
(22) Date of filing: 27.12.2024
(51) Int. Cl.: A61K 9/00, A61K 9/107, A61K 47/42

(54) **OXYGEN CARRIER PHARMACEUTICAL COMPOSITION CONTAINING PERFLUOROCARBON AND CASEIN, AND PREPARATION METHOD THEREFOR**

(30) Priority: 27.12.2023 KR 20230193134
(71) Applicant: Imdpharm Inc., Yeongtong-gu Suwon-si, Gyeonggi-do 16226 (KR)
(72) Inventor: PARK, Young Joon, Seoul 06583 (KR); CHOI, Sook, Seoul 06583 (KR)
(74) Representative: Turner, Craig Robert
(86) International application number: PCT/KR2024/021231
(87) International publication number: WO 2025/143844

(57) **Abstract**

The present invention provides a pharmaceutical composition in which perfluorocarbon is formulated in the form of a nanoemulsion using casein or a salt thereof, and a method for preparing the same. In the pharmaceutical composition of the present invention, perfluorocarbon may be present in the form of stable liquid particles in the nanoemulsion. Therefore, the pharmaceutical composition of the present invention can avoid side effects such as thrombus formation or vascular occlusion and thus can be useful as an artificial oxygen carrier, i.e., a blood substitute.

## Description

### TECHNICAL FIELD

The present invention relates to an oxygen carrier pharmaceutical composition comprising perfluorocarbon and a method for manufacturing the same. More specifically, the present invention relates to a pharmaceutical composition for use as an artificial oxygen carrier, in which perfluorocarbon is formulated in the form of a nanoemulsion using casein or a salt thereof, and a method for preparing the same.

### BACKGROUND ART

Red blood cells in the blood are essential components that play a role in supplying oxygen to tissues and organs in the body and discharging carbon dioxide from the body. Therefore, hemorrhagic shock, which may be occurred due to severe bleeding caused by accidents, trauma, surgery, acute coagulation disorders, and so on, results in death if new blood is not supplied. The oldest, common, and effective method of solving such situations is a transfusion of homologous blood. The transfusion of homologous blood incurs enormous costs for processes such as separating, inspecting, diagnosing, and storing the collected blood, and may cause problems such as infection of diseases and immunological side effects due to different blood types.

One of the alternatives capable of solving these problems is artificial blood. Artificial blood research is being conducted in the fields for developing, e.g., artificial red blood cells that function to transport oxygen, artificial platelets that help blood coagulation, and artificial immunities that prevent coagulation factors and infections. Among them, research on artificial red blood cells (that is, red blood cell substitutes) is actively carried out. Artificial oxygen carriers act as an alternative material that transports oxygen to each tissue of the human body through blood vessels in place of human red blood cells.

Artificial oxygen carriers are very important for national and social health and security, because they pursue oxygen carrying capacity, maintenance of constant colloid osmotic pressure, long-term blood residence time, low toxicity and ease of discharge of material components, and ease of long-term preservation, and can be transfused regardless of blood type. Various blood substitutes are being researched worldwide to solve the problem of insufficient blood supply. In order to supply an oxygen carrier that can replace a red blood cell function, research and development targeting general purpose artificial blood production, such as cell-free and chemically modified hemoglobin, oxygen-affinity polymers, and so on are underway. However, there have been no successful cases yet.

Perfluorocarbons (PFCs), which are chemically and biologically inert, have about 20 times of oxygen solubility in comparison with plasma and thus a high dissolving capacity for oxygen and carbon dioxide to play a role in physically increasing oxygen dissolution in arterial blood. Perfluorocarbons are used in the form of an emulsion because they are not well mixed with water. Since the emulsion contains particulates, it should be injected in low doses to avoid dysfunction of phagocytes in the reticuloendothelial system due to overload.

Fluosol-DA, Oxygent^{™}, and so on have been reported as perfluorocarbon-containing preparations. Fluosol-DA (Green Cross Corp., Osaka, Japan) is a formulation in the form of an emulsion containing perfluorodecalin, perfluorotripropylamine, Pluronic F-68, a phospholipid, etc. However, the use of Fluosol-DA was discontinued due to serious side effects such as hemorrhage, carbon monoxide poisoning, cerebral hypoxia, anemia, angioplasty, microvascular thrombosis and occlusion, and pulmonary toxicity. Oxygent^{™} (Alliance Pharm, USA) is a formulation in the form of an emulsion containing perflubron (perfluorooctyl bromide) and a phospholipid. However, Oxygent^{™} (Alliance Pharm, USA) has been also reported to have side effects such as stroke, flu-like symptoms, thrombus formation, and pulmonary vascular damage.

Therefore, there is a need in the art to develop a perfluorocarbon-containing formulation useful as an oxygen carrier that can avoid side effects such as thrombus formation and vascular occlusion.

### DISCLOSURE

### Technical Problem

The present inventors have carried out various studies to develop an improved formulation containing perfluorocarbons. In particular, the present inventors have carried out various studies to develop a perfluorocarbon-containing emulsion formulation that can avoid side effects such as thrombus formation or vascular occlusion. The present inventors have discovered that, when perfluorocarbon is formulated in the form of a nanoemulsion using casein or a salt thereof for which safety has been ensured, the perfluorocarbon may exist in the form of stable liquid particles within the nanoemulsion.

Therefore, it is an object of the present invention to provide a pharmaceutical composition in the form of a nanoemulsion comprising perfluorocarbon and casein or a salt thereof, which functions as an artificial oxygen carrier.

It is another object of the present invention to provide a method for preparing the pharmaceutical composition in the form of a nanoemulsion.

### Technical Solution

In accordance with an aspect of the present invention, there is provided a pharmaceutical composition in the form of a nanoemulsion for use as an artificial oxygen carrier, which comprises a dispersed phase comprising perfluorocarbon, a surfactant, and casein or a salt thereof in an aqueous continuous phase.

The aqueous continuous phase may be water, a phosphate buffer, or a physiological saline.

The perfluorocarbon may be at least one selected from the group consisting of perfluorooctyl bromide (PFOB), perfluorodecalin (PFD), and perfluorohexane (PFH), preferably perfluorooctyl bromide (PFOB). The perfluorocarbon may be present at a concentration of 10 to 70 w/w%.

The surfactant may be at least one selected from the group consisting of phospholipids, glycerin fatty acid esters, sorbitan fatty acid esters, polyoxyethylene fatty acid esters, propylene glycol fatty acid esters, tocopherol polyethylene glycol succinate (TPGS), macrogol 15 hydrostearate, poloxamer, polyoxyl hydrogenated castor oil, polyoxyl castor oil, polyoxyl cetostearyl esters, and cholesterol. Said surfactant may be a phospholipid, for example, at least one selected from the group consisting of phosphatidylcholine, phosphatidylethanolamine, and lecithin. The surfactant may be present at a concentration of 1 to 10 w/w%.

The casein or a salt thereof may be present at a concentration of 1 to 10 w/w%.

The pharmaceutical composition of the present invention may further comprise at least one viscosity modifier selected from the group consisting of glycerin, mannitol, lactose, trehalose, polyvinyl alcohol, propylene glycol, dextrin, and povidone. The viscosity modifier may be present at a concentration of 1 to 5 w/w%.

In the pharmaceutical composition of the present invention, the dispersed phase may have a diameter of 300 nm or less.

In accordance with another aspect of the present invention, there is provided a method for preparing said pharmaceutical composition, the method comprising: (a) adding casein or a salt thereof, a surfactant, optionally a viscosity modifier, and perfluorocarbon to an aqueous continuous phase, followed by homogenization thereof, and (b) dispersing the dispersion obtained in step (a) with a high-pressure homogenizer to form a nanoemulsion.

### ADVANTAGEOUS EFFECTS

It has been found by the present invention that, when perfluorocarbon is formulated in the form of a nanoemulsion using casein or a salt thereof, the perfluorocarbon may exist in the form of stable liquid particles within the nanoemulsion. Therefore, the pharmaceutical composition in the form of a nanoemulsion according to the present invention can avoid side effects such as thrombus formation or vascular occlusion, and thus it can be usefully used as an artificial oxygen carrier, that is, a blood substitute.

### DESCRIPTION OF DRAWINGS

FIG. 1 shows a Cryo-TEM image of the nanoemulsion containing casein at a concentration of 2.5 w/w% (No. 12 of Example 2).
FIG. 2 shows a Cryo-TEM image of the nanoemulsion containing no casein (No. 17 of Example 2).

### BEST MODE

The present invention provides a pharmaceutical composition in the form of a nanoemulsion for use as an artificial oxygen carrier, which comprises a dispersed phase comprising perfluorocarbon, a surfactant, and casein or a salt thereof in an aqueous continuous phase.

In the pharmaceutical composition of the present invention, the aqueous continuous phase may be water (e.g., water for injection), a phosphate buffer, or a physiological saline. If necessary, the aqueous continuous phase may further comprises a viscosity modifier such as glycerin.

The perfluorocarbon includes various perfluorocarbons known as artificial oxygen carriers. For example, the perfluorocarbon may be at least one selected from the group consisting of perfluorooctyl bromide (PFOB) [also known as perflubron], perfluorodecalin (PFD), and perfluorohexane (PFH). In an embodiment, the perfluorocarbon may be perfluorooctyl bromide (PFOB). The perfluorocarbon may be used at a sufficient concentration to exhibit efficacy as an artificial oxygen carrier. For example, the perfluorocarbon may be present at a concentration of 10 to 70 w/w%, preferably 20 to 60 w/w%.

The surfactant acts as an emulsifier for forming a nanoemulsion. The surfactant includes, without limitation, an emulsifier used in a perfluorocarbon-containing formulation. For example, the surfactant may be at least one selected from the group consisting of phospholipids, glycerin fatty acid esters, sorbitan fatty acid esters, polyoxyethylene fatty acid esters (e.g., polyoxyethylene 20 sorbitan monolaurate, polyoxyethylene 80 sorbitan monooleate, etc.), propylene glycol fatty acid esters, tocopherol polyethylene glycol succinate (TPGS), macrogol 15 hydrostearate, poloxamer, polyoxyl hydrogenated castor oil, polyoxyl castor oil, polyoxyl cetostearyl esters, and cholesterol. Preferably, the surfactant may be a phospholipid. In an embodiment, the phospholipid may be at least one selected from the group consisting of phosphatidylcholine, phosphatidylethanolamine, and lecithin. The surfactant may be present at a concentration of 1 to 10 w/w%, preferably 1 to 5 w/w%.

In the pharmaceutical composition of the present invention, casein or a salt thereof (e.g., sodium casein) functions to allow perfluorocarbon to exist in the form of stable liquid particles in the nanoemulsion. The casein or a salt thereof is presumed to encapsulate perfluorocarbon liquid particles to form a core-shell structure or to modify the surface of the perfluorocarbon liquid particles (surface modification). The casein or a salt thereof may be used in an amount such that perfluorocarbon can exist in the form of stable liquid particles in the nanoemulsion. For example, the casein or a salt thereof may be present at a concentration of 1 to 10 w/w%, preferably 1 to 5 w/w %.

The pharmaceutical composition of the present invention may include pharmaceutically acceptable excipients conventionally used in perfluorocarbon-containing formulations. For example, the pharmaceutical composition may further comprise at least one viscosity modifier selected from the group consisting of glycerin, mannitol, lactose, trehalose, polyvinyl alcohol, propylene glycol, dextrin, and povidone. The viscosity modifier may be present at a concentration of 1 to 5 w/w%.

The pharmaceutical composition of the present invention is in the form of a nanoemulsion, and, for example, the dispersed phase (i.e., perfluorocarbon liquid particles) may have a diameter of 300 nm or less, preferably 70 to 200 nm.

The present invention includes a method for preparing said pharmaceutical composition. For example, the method of the present invention may comprise (a) adding casein or a salt thereof, a surfactant, optionally a viscosity modifier, and perfluorocarbon to an aqueous continuous phase, followed by homogenization thereof, and (b) dispersing the dispersion obtained in step (a) with a high-pressure homogenizer to form a nanoemulsion.

In the method of the present invention, the aqueous continuous phase, the perfluorocarbon, the surfactant, the viscosity modifier, etc. are as described in relation to the pharmaceutical composition. Homogenizing in step (a) and dispersing in step (b) may be carried out using a homogenizer and a high-pressure homogenizer according to conventional methods.

The present invention will be described in further detail with reference to the following examples. However, these examples are for illustrative purposes only and are not intended to limit the scope of the present invention.

### Example 1: Preparation and Evaluation of Nanoemulsions

Emulsions were prepared according to the ingredients and contents of Table 1 below. Specifically, after sodium casein was dissolved in distilled water, lecithin, glycerin, and perfluorooctyl bromide (PFOB) were added thereto, followed by homogenization thereof. The obtained dispersions (dispersions in the form of pre-emulsion) were dispersed with a high-pressure homogenizer to prepare the nanoemulsions.

The particle size, zeta potential, and maximum oxygen-release concentration (Oxygen Release Cₘₐₓ) of the obtained nanoemulsions were measured, respectively. The particle size and zeta potential were measured using an ELSZ-2000 instrument (Otsuka Electronics Korea).

The maximum oxygen-release concentrations were measured under the following conditions.
- Sample amount: 1.5 mL
- Flask volume: 50 mL
- Sample Oxygenation: 1 hour
- Medium: 50 mL of nitrogen-substituted pH 7.4 PBS buffer (oxygen concentration: 0.01 mg/L)
- Stirring rate: 200 rpm
- Evaluation item: dissolved oxygen (mg/L)

After blocking the air with a rubber cap, each sample was oxygenated under stirring at 200 rpm for 1 hour. After blocking the air with a rubber cap, 50 mL of pH 7.4 PBS buffer was nitrogen-substituted under stirring at 200 rpm for 1 hour so that the oxygen concentration was 0.01 mg/L or less. 1.5 mL of each sample was added to the 50 mL of pH 7.4 PBS buffer and the oxygen-release concentration was checked while stirring at 200 rpm.

The results of measuring the particle size, zeta potential, and maximum oxygen-release concentration as described above are shown in Table 1 below.

**Table 1**

| No. | Sodium casein | Lecithin | PFOB | Glycerin | Particle size | Zeta potential | maximum oxygen-release concentration |
|---|---|---|---|---|---|---|---|
| | %, w/w | %, w/w | %, w/w | %, w/w | nm | mV | mg/L |
| 1 | 1.50 | 2.50 | 40.00 | 2.50 | 363.89 | -11.56 | 8.89 |
| 2 | 2.50 | 2.50 | 40.00 | 2.00 | 145.56 | -23.67 | 10.23 |
| 3 | 2.50 | 2.00 | 10.00 | 2.50 | 156.72 | -18.28 | 4.34 |
| 4 | 2.00 | 2.25 | 25.00 | 1.75 | 178.50 | -12.56 | 6.45 |
| 5 | 1.50 | 2.50 | 10.00 | 2.50 | 346.78 | -12.67 | 3.82 |
| 6 | 2.00 | 2.75 | 25.00 | 2.25 | 167.56 | -18.76 | 6.01 |
| 7 | 2.50 | 2.00 | 40.00 | 2.50 | 163.61 | -17.85 | 9.82 |
| 8 | 2.00 | 2.25 | 25.00 | 2.25 | 176.89 | -19.23 | 2.02 |
| 9 | 1.50 | 2.00 | 10.00 | 2.00 | 423.78 | -12.64 | 3.01 |
| 10 | 2.00 | 2.25 | 55.00 | 2.25 | 201.67 | -19.34 | 11.21 |
| 11 | 2.50 | 2.00 | 40.00 | 2.00 | 126.78 | -18.56 | 10.85 |
| 12 | 1.50 | 2.00 | 40.00 | 2.50 | 576.67 | -9.87 | 6.38 |
| 13 | 3.00 | 2.25 | 25.00 | 2.25 | 115.20 | -38.56 | 7.89 |
| 14 | 2.00 | 2.25 | 25.00 | 2.75 | 167.45 | -17.12 | 5.16 |
| 15 | 2.00 | 2.25 | 25.00 | 2.25 | 167.34 | -16.34 | 5.03 |
| 16 | 2.50 | 2.50 | 10.00 | 2.00 | 138.45 | -31.67 | 5.45 |
| 17 | 2.50 | 2.50 | 40.00 | 2.50 | 121.34 | -26.35 | 11.01 |
| 18 | 2.50 | 2.00 | 10.00 | 2.00 | 127.67 | -18.45 | 4.87 |
| 19 | 2.00 | 2.25 | 25.00 | 2.25 | 169.34 | -16.34 | 6.19 |
| 20 | 1.50 | 2.50 | 10.00 | 2.00 | 317.56 | -9.65 | 3.38 |
| 21 | 1.50 | 2.50 | 40.00 | 2.00 | 335.63 | -9.65 | 7.37 |
| 22 | 1.50 | 2.00 | 10.00 | 2.50 | 389.34 | -8.78 | 4.02 |
| 23 | 2.50 | 2.50 | 10.00 | 2.50 | 145.76 | -24.32 | 5.38 |
| 24 | 2.00 | 2.225 | 25.00 | 2.25 | 169.34 | -16.34 | 5.99 |
| 25 | 2.00 | 2.25 | 25.00 | 2.25 | 169.34 | -16.34 | 5.99 |
| 26 | 1.50 | 2.00 | 40.00 | 2.00 | 347.8 | -8.56 | 6.67 |
| 27 | 2.00 | 1.75 | 25.00 | 2.25 | 423.7 | -12.67 | 6.01 |
| 28 | 1.00 | 2.25 | 25.00 | 2.25 | 587.34 | -6.45 | 4.99 |

As the concentration of casein increased, the particle size tended to decrease, and as the concentration of lecithin increased, the particle size also tended to decrease. In terms of particle size, no significant change was observed according to the ratio of PFOB and glycerin.

As the concentration of casein increased, the zeta potential tended to decrease. In terms of zeta potential, no significant change was observed according to the concentrations of PFOB, lecithin, and glycerin.

As the concentrations of casein and PFOB increased, the maximum oxygen-release concentration tended to increase. In terms of maximum oxygen-release concentration, no significant change was observed according to the concentration ratio of lecithin and glycerin.

### Example 2: Preparation and Evaluation of Nanoemulsions

In order to select the optimal composition based on the results obtained in Example 1, nanoemulsions were prepared according to the ingredients and contents of of Table 2 with a concentration of perfluorooctyl bromide (PFOB) of 40 w/w%. In addition, the particle size and zeta potential of each nanoemulsion were measured in the same manner as in Example 1, and the results are shown in Table 2 below.

**Table 2**

| No. | PFOB | Sodium casein | Lecithin | Glycerin | Distilled water | Particle size | Zeta potential |
|---|---|---|---|---|---|---|---|
| | %, w/w | | | | | (nm) | (mV) |
| 1 | 40.00 | 1.01 | 1.81 | 3.99 | 53.19 | 170.1±2.3 | 0.45±1.8 |
| 2 | | 3.99 | 1.81 | 1.01 | 53.19 | 157.2±1.6 | -9.23±0.95 |
| 3 | | 2.50 | 3.00 | 0.00 | 54.50 | 146.3±9.4 | -7.73±0.27 |
| 4 | | 2.50 | 3.00 | 2.50 | 52.00 | 157.2±23.9 | -7.83±0.40 |
| 5 | | 3.99 | 1.81 | 3.99 | 50.21 | 179.1±10.3 | -7.57±0.25 |
| 6 | | 2.50 | 3.00 | 2.50 | 52.00 | 138.1±1.7 | -8.35±1.00 |
| 7 | | 1.01 | 1.81 | 1.01 | 56.17 | 175.8±2.0 | -15.19±0.45 |
| 8 | | 2.50 | 1.00 | 2.50 | 54.00 | 184.8±2.3 | -10.96±1.24 |
| 9 | | 2.50 | 3.00 | 2.50 | 52.00 | 130.7±0.4 | -6.53±1.70 |
| 10 | | 1.01 | 4.19 | 1.01 | 53.79 | 125.4±1.1 | -3.39±0.35 |
| 11 | | 1.01 | 4.19 | 3.99 | 50.81 | 133.3±1.4 | -4.34±1.03 |
| 12 | | 2.50 | 3.00 | 2.50 | 52.00 | 161.5±2.3 | -5.97±1.40 |
| 13 | | 2.50 | 3.00 | 5.00 | 49.50 | 128.9±0.8 | -7.97±0.85 |
| 14 | | 3.99 | 4.19 | 1.01 | 50.81 | 149.0±17.3 | -6.99±0.99 |
| 15 | | 5.00 | 3.00 | 2.50 | 49.50 | 156.4±16.6 | -7.25±0.85 |
| 16 | | 3.99 | 4.19 | 3.99 | 47.83 | 126.0±0.3 | -2.30±0.78 |
| 17 | | 0.00 | 3.00 | 2.50 | 54.50 | 148.1±2.7 | 4.25±0.37 |
| 18 | | 2.50 | 3.00 | 2.50 | 52.00 | 136.7±1.4 | -6.73±1.18 |
| 19 | | 2.50 | 5.00 | 2.50 | 50.00 | 97.8±2.9 | -6.34±0.99 |
| 20 | | 2.50 | 3.00 | 2.50 | 52.00 | 151.0±0.6 | -7.61±1.13 |

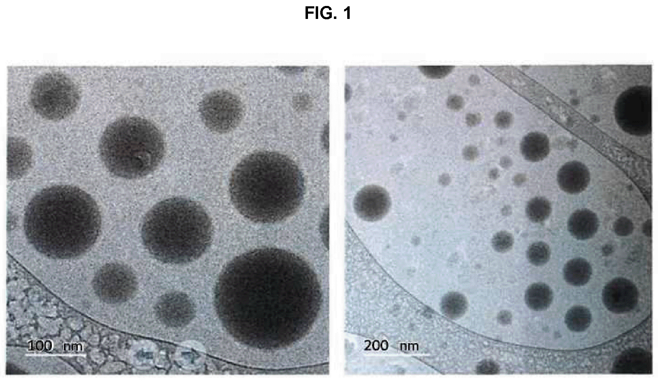

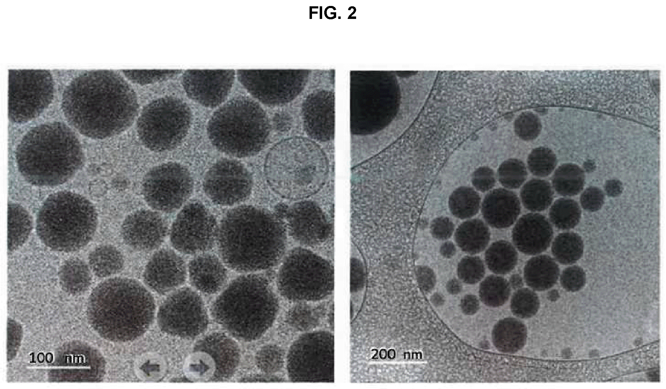

The results of measuring the particle structure using cryo-TEM for the nanoemulsion containing casein at a concentration of 2.5 w/w% (No. 12) and the nanoemulsion containing no casein (No. 17) are shown in FIGs. 1 and 2, respectively. While the nanoemulsion containing casein showed a particle shape close to the spherical shape (FIG. 1), the nanoemulsion containing no casein showed the shapes that was distorted or was stuck together and pressed with very close spacing between particles (FIG. 2). Therefore, it can be confirmed that the nanoemulsions containing casein exhibit excellent stability.

### Example 3: Preparation of Nanoemulsions

Emulsions were prepared according to the ingredients and contents of Table 3 below. Specifically, after sodium casein was dissolved in distilled water, a surfactant and/or a viscosity modifier (lecithin, TPGS, poloxamer 407, Kolliphor HS 15, glycerin, and/or mannitol), and perfluorocarbon [perfluorooctyl bromide (PFOB) and/or perfluorodecalin (PFD)] were added thereto, followed by homogenization thereof. The obtained dispersions (dispersions in the form of pre-emulsion) were dispersed with a high-pressure homogenizer to prepare the nanoemulsions.

**Table 3**

| No. | PFOB | PFD | Sodium casein | Lecithin | TPGS | Poloxa mer 407 | Kollipho r HS 15 | Glycerin | Mannitol | Distilled water |
|---|---|---|---|---|---|---|---|---|---|---|
| 1 | 20 | | 2 | 2 | | 2 | | | | 74 |
| 2 | 25 | | 10 | 2 | 3 | | | 5 | | 55 |
| 3 | 25 | | 2.5 | 2 | | 1 | | 1.5 | | 68 |
| 4 | 30 | 10 | 4 | 3 | 1 | | | 2 | 2 | 48 |
| 5 | 30 | 10 | 1 | 2 | | | 2 | | | 55 |
| 6 | 40 | | 1 | 2 | | | | | | 57 |
| 7 | 40 | | 2 | 2 | | | | 1.5 | | 54.5 |
| 8 | 40 | | 3 | 2 | | | | 1.5 | | 53.5 |
| 9 | 40 | | 5 | 2 | | | | 3 | | 50 |
| 10 | 40 | | 7 | 2 | | | | 4 | | 47 |
| 11 | 40 | | 8 | 2 | | | | 3 | | 47 |
| 12 | 45 | | 2 | 1 | | 1 | | | | 51 |
| 13 | 45 | | 4 | 3 | | | | 2 | | 46 |
| 14 | 45 | | 2 | 8 | | | | 1.5 | | 43.5 |
| 15 | 45 | | 4 | 2 | 2 | | | 4 | | 43 |
| 16 | 45 | | 3 | 5 | | | | 1.5 | | 45.5 |
| 17 | 25 | | 10 | 2 | 3 | | | 5 | | 55 |
| 18 | 60 | | 3 | 2 | | | | 1.5 | | 33.5 |
| 19 | 60 | | 1 | | | 3 | | 1.5 | | 34.5 |
| 20 | 60 | | 2 | 2 | 2.5 | | | 1.5 | | 32 |
| 21 | 60 | | 3 | | 2 | | | 5 | | 30 |
| 22 | 60 | | 2 | | | 2 | | 2 | | 34 |
| 23 | 70 | | 2 | 2 | | | | 1 | | 25 |

## Claims

1. A pharmaceutical composition in the form of a nanoemulsion for use as an artificial oxygen carrier, which comprises a dispersed phase comprising perfluorocarbon, a surfactant, and casein or a salt thereof in an aqueous continuous phase.

2. The pharmaceutical composition as claimed in claim 1, wherein the aqueous continuous phase is water, a phosphate buffer, or a physiological saline.

3. The pharmaceutical composition as claimed in claim 1, wherein perfluorocarbon is at least one selected from the group consisting of perfluorooctyl bromide (PFOB), perfluorodecalin (PFD), and perfluorohexane (PFH).

4. The pharmaceutical composition as claimed in claim 3, wherein perfluorocarbon is perfluorooctyl bromide (PFOB).

5. The pharmaceutical composition as claimed in claim 1, wherein the perfluorocarbon is present at a concentration of 10 to 70 w/w%.

6. The pharmaceutical composition as claimed in claim 1, wherein the surfactant is at least one selected from the group consisting of phospholipids, glycerin fatty acid esters, sorbitan fatty acid esters, polyoxyethylene fatty acid esters, propylene glycol fatty acid esters, tocopherol polyethylene glycol succinate (TPGS), macrogol 15 hydrostearate, poloxamer, polyoxyl hydrogenated castor oil, polyoxyl castor oil, polyoxyl cetostearyl esters, and cholesterol.

7. The pharmaceutical composition as claimed in claim 6, wherein the surfactant is a phospholipid.

8. The pharmaceutical composition as claimed in claim 7, wherein the phospholipid is at least one selected from the group consisting of phosphatidylcholine, phosphatidylethanolamine, and lecithin.

9. The pharmaceutical composition as claimed in claim 1, wherein the surfactant is present at a concentration of 1 to 10 w/w%.

10. The pharmaceutical composition as claimed in claim 1, wherein the casein or a salt thereof is present at a concentration of 1 to 10 w/w%.

11. The pharmaceutical composition as claimed in claim 1, further comprising at least one viscosity modifier selected from the group consisting of glycerin, mannitol, lactose, trehalose, polyvinyl alcohol, propylene glycol, dextrin, and povidone.

12. The pharmaceutical composition as claimed in claim 11, wherein the viscosity modifier is present at a concentration of 1 to 5 w/w%.

13. The pharmaceutical composition as claimed in any one of claims 1 to 12, wherein the dispersed phase has a diameter of 300 nm or less.

14. A method for preparing the pharmaceutical composition as claimed in any one of claims 1 to 12, the method comprising:
(a) adding casein or a salt thereof, a surfactant, optionally a viscosity modifier, and perfluorocarbon to an aqueous continuous phase, followed by homogenization thereof, and
(b) dispersing the dispersion obtained in step (a) with a high-pressure homogenizer to form a nanoemulsion.
